Europäisches Patentamt

European Patent Office

Office européen des brevets

(19)

(11) Veröffentlichungsnummer: **0 273 378**
**B1**

(12) **EUROPÄISCHE PATENTSCHRIFT**

(45) Veröffentlichungstag der Patentschrift:
26.09.90

(51) Int. Cl.⁵: **C07C 49/255, C07C 45/61**

(21) Anmeldenummer: 87119096.3

(22) Anmeldetag: 23.12.87

(54) Acetylketendialkylacetale und Verfahren zu ihrer Herstellung.

(30) Priorität: 30.12.86 DE 3644661

(43) Veröffentlichungstag der Anmeldung:
06.07.88 Patentblatt 88/27

(45) Bekanntmachung des Hinweises auf die Patenterteilung:
26.09.90 Patentblatt 90/39

(84) Benannte Vertragsstaaten:
BE CH DE FR GB IT LI NL

(56) Entgegenhaltungen:
CHEMICAL ABSTRACTS, Band 55, Nr. 23, 13. November 1961, Columbus, Ohio, USA A.N. NESMEYANOV et al. "Some reactions of methyl Beta, Beta-dichlorvinyl ketone" Spalten 23335, 23336
THE JOURNAL OF THE AMERICAN CHEMICAL SOCIETY, Band LXXIV, 1952, Easton, PA, Mack printing Company, USA S.M. MC ELVAIN et al.: "Ketene Acetales XXIX. The Mechanism of the Reaction of Keten Acetal with Various Halides" Seiten 2662-2667

(73) Patentinhaber: HOECHST AKTIENGESELLSCHAFT, Postfach 80 03 20, D-6230 Frankfurt am Main 80(DE)

(72) Erfinder: Günther, Klaus, Dr., Waldstrasse 5, D-6239 Eppstein/Taunus(DE)
Erfinder: Mau, Günter, Dr., Im Stückes 32, D-6233 Kelkheim (Taunus)(DE)

**Beschreibung**

Die Erfindung betrifft Acetylketendialkylacetale und ein Verfahren zu ihrer Herstellung. Nach Literaturangaben wird Acetylketendiethylacetal $CH_3-CO-CH=C(OC_2H_5)_2$ durch Umsetzung von Ketendiethylacetal mit Acetylchlorid hergestellt, wobei jedoch erheblicher Mengen Nebenprodukte entstehen (Mc Elvain, Mc Shane, J.Am.Chem.Soc. 74, 1952, Seiten 2662, 2666). Jedoch ist die Ausgangssubstanz Ketendiethylacetal schwer zugänglich; seine Synthese weist viele Reaktionsstufen auf, die meist mit unbefriedigender Ausbeute verlaufen.

Demgemäß ist dieses Verfahren zur Herstellung von Acetylketendiethylacetal im größeren Maßstab wenig geeignet. Weiterhin ist in der Literatur nur noch die Existenz von Acetylketendimethylacetal erwähnt. Acetylketendialkylacetale die höhere Alkylgruppen als Methyl und Ethyl enthalten. werden nicht erwähnt.

Wir haben nun ein einfaches Verfahren gefunden, das es gestattet, Acetylketendialkylacetale $CH_3-CO-CH=C(OR)_2$ mit einem weiten Bereich von Alkylgruppen zu gewinnen.

Ein Gegenstand der Erfindung ist ein Verfahren zur Herstellung von Acetylketendialkylacetalen der Formel $CH_3CO-CH=C(OR^2)_2$, wobei $R^2$ ein Alkylrest mit 1 bis 12 C-Atomen sein kann, dadurch gekennzeichnet, daß man einen Acetessigsäurealkylester der Formel $CH_3CO-CH_2COOR^1$ in Gegenwart eines sauren Katalysators mit einem Alkohol der Formel $R^2OH$ umsetzt, wobei $R^2$ die angegebene Bedeutung hat und $R^2OH$ entweder mit $R^1OH$ identisch ist oder höher siedet als $R^1OH$.

Ein weiterer Gegenstand der Erfindung sind Acetylketendialkylacetale der Formel $CH_3CO-CH=C(OR^2)_2$, wobei $R^2$ ein Alkylrest mit 3 bis 12, vorzugsweise 3 bis 8 C-Atomen ist.

Überraschenderweise bildet sich bei dem erfindungsgemäßen Verfahren Acetylketendialkylacetal, ohne daß größere Anteile von unerwünschten, nicht verwertbaren Komponenten entstehen.

Bei der Umsetzung von Acetessigsäurealkylester $CH_3COCH_2COOR^1$ mit einem Alkohol $R^2OH$, der höher siedet als der freiwerdende Alkohol $R^1OH$, findet als Parallelreaktion eine Umesterung statt, dh. es bildet sich neben dem gewünschten Acetylketendialkylacetal $CH_3COCH=C(OR^2)_2$ eine etwa gleichgroße Menge Acetessigsäurealkylester $CH_3COCH_2COOR^2$, der die Alkylgruppe des als Reaktionspartner verwendeten Alkohols $R^2OH$ enthält.

Der bei dieser Umsetzung freiwerdende leichter siedende Alkohol $R^1OH$ destilliert vollständig ab, so daß schließlich das Reaktionsgemisch im wesentlichen nur das Acetylketendialkylacetal, $CH_3COCH=C(OR^2)_2$, den durch Umesterung gebildeten Acetessigsäurealkylester $CH_3COCH_2COOR^2$, sowie den im Überschuß eingesetzten Alkohol $R^2OH$ enthält. Das gewonnene Reaktionsgemisch läßt sich durch fraktionierte Destillation leicht trennen, wobei das Acetylketendialkylacetal als am höchsten siedende Komponente zuletzt überdestilliert.

Vorzugsweise geht man bei der Reaktion vom Methyl- oder Ethylester der Acetessigsäure aus; beide Verbindungen sind leicht verfügbar, insbesondere der Methylester. Der für die Umsetzung erforderliche Alkohol $R^2OH$ wird vorzugsweise im Überschuß eingesetzt. Falls $R^2OH$ mit $R^1OH$ identisch ist, setzt man daher vorzugsweise 2,5 bis 5 Mol Alkohol pro Mol Acetessigsäureester ein; falls $R^2OH$ höher siedet als $R^1OH$, setzt man vorzugsweise 1,5 bis 5 Mol Alkohol $R^2OH$ pro Mol Acetessigsäureester $CH_3COCH_2COOR^1$ ein.

Als Alkohole $R^2OH$ kommen gesättigte primäre und sekundäre, vorzugsweise primäre Alkohole mit 1 bis 12 C-Atomen, vorzugsweise 3 bis 6 C-Atomen in Frage, wobei die C-Kette geradkettig oder verzweigt sein kann.

Die Reaktionstemperatur sollte etwa 110-170 °C betragen. vorzugsweise 130-160 °C, um eine hinreichend hohe Reaktionsgeschwindigkeit zu erzielen. Bei Einsatz der relativ niedrig siedenden Alkohole $R^2OH$ mit 1 bis 3 C-Atomen hat es sich als zweckmäßig erwiesen, die erforderliche Reaktionstemperatur von mindestens 110 °C dadurch zu erreichen, daß man die Reaktion unter geeignet erhöhtem Druck durchführt.

Als Katalysatoren benutzt man saure Komponenten. Bevorzugt kommen Protonensäuren, wie Schwefelsäure, p-Toluolsulfonsäure oder Phosphorsäure zum Einsatz. Auch saure Ionenaustauscher sowie Molekularsiebe sind geeignet. Der Konzentrationsbereich für den eingesetzten Katalysator liegt bei 0,005 bis 5 Gew.-%. Als sehr günstig hat sich bei Verwendung von konzentrierter Schwefelsäure eine Konzentration von 0,05 bis 0,2 Gew.-% erwiesen.

Im technischen Maßstab kann die Herstellung der Acetylketendialkylacetale nach dem erfindungsgemäßen Verfahren sowohl diskontinuierlich im Chargenbetrieb als auch kontinuierlich in einer geeigneten Apparateanordnung erfolgen.

Bei der diskontinuierlichen Gewinnung des Acetylketendialkylacetals aus dem bevorzugt eingesetzten Acetessigsäuremethylester wird in einer ersten Stufe dieser Ester mit überschüssigem Alkohol $R^2OH$ umgesetzt, wobei das freiwerdende leichtflüchtige Methanol gesondert gewonnen wird, und anschließend der Überschußalkohol $R^2OH$ zusammen mit dem durch Umesterung gebildeten Acetessigsäurealkylester $CH_3COCH_2COOR^2$ unter Vakuum abdestilliert, wobei das in dieser Stufe gebildete Acetylketendialkylacetal zurückbleibt. In einer zweiten Stufe läßt man den abdestillierten Acetessigsäurealkylester mit überschüssigem Alkohol $R^2OH$ wiederum unter Reaktionsbedingungen zu Acetylketendialkylacetal reagieren und destilliert danach den Überschußalkohol $R^2OH$ zusammen mit dem unumgesetzten Acetessigsäurealkylester $CH_3COCH_2COOR^2$ unter Vakuum ab, wobei das in der 2. Stufe gebildete Acetylketendialkylacetal zurückbleibt. Zur Verbesserung der Ausbeute, bezogen auf den eingesetzten Acetessigsäuremethylester, kann man eventuell mit dem nach der zweiten Stufe abdestillierten Acetessigsäurealkylester-Überschußalkohol-Gemisch eine nochmalige Umsetzung durchführen. Das in den einzelnen Schritten gebildete Acetylketendialkylacetal wird vereinigt und gemeinsam durch Vakuumdestillation gereinigt.

Die kontinuierliche Herstellung des Acetylketendialkylacetals kann in einer dafür geeigneten Apparatur auf recht elegante Weise erfolgen. In einen Reaktor speist man Acetessigsäuremethylester zusammen mit überschüssigem Alkohol R$^2$OH und Katalysator ein. Aus dem Reaktor wird Methanol dampfförmig, und das Reaktionsgemisch, bestehend aus Acetylketendialkylacetal CH$_3$COCH=C(OR$^2$)$_2$, Acetessigsäurealkylester CH$_3$COCH$_2$COOR$^2$ und Alkohol R$^2$OH flüssig abgezogen. Das Reaktionsgemisch gelangt in eine Destillationskolonne, bei der über Kopf der überschüssige Alkohol R$^2$OH mit nicht umgesetztem Acetessigsäurealkylester abdestilliert. Das Kopfprodukt wird in den Reaktor zurückgeführt. Aus dem Sumpf der Kolonne entnimmt man das unreine Acetylketendialkylacetal und führt es einer weiteren Kolonne zu, in der es über Kopf als Reinprodukt abdestilliert. Im Sumpf dieser Kolonne reichern sich der Katalysator und hochsiedende Rückstände an, die einer Entsorgungsanlage zugeführt werden.

Die Acetylketendialkylacetale sind Verbindungen, die als Ausgangsprodukte für Synthesen vielfältig eingesetzt werden können. Durch ihre Konstitution sind sie für Cyclisierungsreaktionen besonders geeignet. Es ist bekannt, daß Acetylketendimethylacetal und Acetylketendiethylacetal sich bei bestimmten Temperaturen als 1-Hydroxyvinylketenacetale verhalten und zum Beispiel an definierten Positionen substituierte Anthrachinone ergeben, die als Naturfarbstoffe von großer Bedeutung sind (J.Chem.Soc.Perkin Trans.I, 1976, 17, 1872-56). Analog gilt dies für die Homologen mit längeren Alkylresten. Die Möglichkeit, durch das erfindungsgemäße Verfahren Acetylketendialkylacetale im größeren Umfang günstig herstellen zu können, eröffnet eine Vielzahl von synthetischen Möglichkeiten.

Die folgenden Beispiele sollen die Erfindung erläutern.

Beispiel 1

In einem 1 l-Glaskolben mit aufgesetztem Kühler und Rührwerk ließ man 174 g Acetessigsäuremethylester, 528 g Pentanol (Mischung aus ca. 85 Gew. % Pentan-1-ol und ca. 15 Gew. % 1-Methyl-butan-1-ol) und 3,5 g Schwefelsäure bei 130-140°C ca. 2,5 Stunden reagieren. Der Kühler war auf 90°C thermostatisiert, so daß niedrig siedende Komponenten abdestillieren konnten. Nach Beendigung der Umsetzung enthielt das Reaktionsgemisch 136 g Acetessigsäurepentylester, 133 g Acetylketendipentylacetal, 344 g Pentanol, 5 g Acetessigsäuremethylester, 3,5 g Schwefelsäure und 26 g unbekannte Substanzen.

Aus dem Reaktionsgemisch wurde durch fraktionierte Destillation Acetylketendipentylacetal CH$_3$COCH$_2$=C(OC$_5$H$_{11}$)$_2$ mit einer Reinheit von über 99 Gew.-% gewonnen (Kp 120°C bei 3 mbar, d$_4^{20}$ =0,9200, n$_D^{20}$ =1,4548).

Beispiel 2

Aus einem wie in Beispiel 1 hergestellten Reaktionsgemisch wurden von dem gebildeten Acetylketendipentylacetal im Vakuum das überschüssige Pentanol sowie der entstandene Acetessigsäurepentylester abdestilliert. Das Destillat enthielt 1210 g Pentanol , 671 g Acetessigsäurepentylester und 24 g sonstige Komponenten. Nach Zusatz von 2 g Schwefelsäure ließ man das Destillat in der in Beispiel 1 beschriebenen Apparatur bei 130 bis 140 °C ca. 3 Stunden lang reagieren. Danach bestand das Reaktionsgemisch aus 1054 g Pentanol, 328 g Acetessigsäurepentylester, 360 g Acetylketendipentylacetal und 38 g sonstigen Komponenten. Bei der Reaktion fielen 127 g Destillat an, davon waren 26 Gew.-% Wasser.

Beispiel 3

In der in Beispiel 1 beschriebenen Apparatur ließ man 1160 g Acetessigsäuremethylester, 2960 g Butan-1-ol und 4,4 g Schwefelsäure bei ca. 120°C reagieren. Nach 3,5 Stunden enthielt das Reaktionsgemisch 1388 g Butan-1-ol, 40 g Acetessigsäuremethylester, 1003 g Acetessigsäurebutylester, 588 g Acetylketendibutylacetal und 58 g sonstige Komponenten. Bei der Reaktion fielen 1048 g Destillat an, das im wesentlichen aus Methanol, Butanol und Wasser bestand. Aus dem Gemisch ließ sich durch fraktionierte Destillation Acetylketendibutylacetal mit einer Reinheit von über 99 % gewinnen (Kp 140-142°C bei 20 mbar, d$_4^{20}$=0,9390; n$_D^{20}$=1,4539; Elementaranalyse (in Gew. %) berechnet: 67,3 % C; 10,3 % H; 22,4 % 0; gefunden: 67,2 % C; 10,4 % H; 22,2 % 0).

Beispiel 4

In der in Beispiel 1 beschriebenen Apparatur ließ man 232 g Acetessigsäuremethylester, 714 g Pentan-1-ol und 1 g Schwefelsäure bei 130 bis 140°C reagieren. Nach 2,5 Stunden enthielt das Reaktionsgemisch 487 g Pentan-1-ol, 188 g Acetessigsäurepentylester, 173 g Acetylketendipentylacetal und 14 g sonstige Komponenten. Bei der Reaktion fielen 85 g Destillat an, das im wesentlichen aus Methanol, Aceton und Wasser bestand. Aus dem Gemisch ließ sich durch fraktionierte Destillation Acetylketendipentylacetal mit einer Reinheit von über 99 % gewinnen (Kp 145-148°C bei 10 mbar; d$_4^{20}$= 0,9244; n$_D^{20}$= 1,4564; Elementaranalyse (in Gew. %) berechnet: 69,4 % C; 10,7 % H; 19,8 % O; gefunden 69,4 % C, 10,6 % H; 19,8 % O).

Beispiel 5

In der in Beispiel 1 beschriebenen Apparatur ließ man 332 g Acetessigsäuremethylester, 816 g Hexan-1-ol und 1,3 g Schwefelsäure bei 140 bis 150°C reagieren. Nach 3,5 Stunden enthielt das Reaktionsgemisch 520 g Hexan-1-ol, 260 g Acetessigsäurehexylester, 177 g Acetylketendihexylacetal und 83 g sonsti-

ge Komponenten. Bei der Reaktion fielen 109 g Destillat an, das im wesentlichen aus Methanol, Aceton und Wasser bestand. Aus dem Gemisch ließ sich durch fraktionierte Destillation Acetylketendihexylacetal mit einer Reinheit von über 99 % gewinnen (Kp 151 - 153°C bei 3mbar, $d_4^{20} = 0{,}9177$; $n_D^{20} = 1{,}4588$; Elementaranalyse (in Gew. %) berechnet: 71,1 % C; 11,1 % H; 17,8 % O; gefunden: 71,1 % C; 11,0 % H; 17,8 % O).

Beispiel 6

In der in Beispiel 1 beschriebenen Apparatur ließ man 332 g Acetessigsäuremethylester, 780 g Heptan-1-ol und 1 g Schwefelsäure bei 150°C reagieren. Nach 3,5 Stunden enthielt das Reaktionsgemisch 488 g Heptan-1-ol, 279 g Acetessigsäureheptylester, 159 g Acetylketendiheptylacetal und 70 g sonstige Komponenten. Bei der Reaktion fielen 117 g Destillat an, das im wesentlichen aus Methanol, Aceton und Wasser bestand.

Beispiel 7

In der in Beispiel 1 beschriebenen Apparatur ließ man 116 g Acetessigsäuremethylester, 528 g 2-Ethyl-hexan-1-ol und 0,6 g Schwefelsäure bei 150°C reagieren. Nach 3,5 Stunden enthielt das Reaktionsgemisch 417 g 2-Ethyl-hexan-1-ol, 89 g Acetessigsäureethylhexylester, 65 g Acetylketendiethylhexylacetal und 24 g sonstige Komponenten. Bei der Reaktion fielen 50 g Destillat an, das im wesentlichen aus Methanol, Aceton und Wasser bestand.

## Patentansprüche

1. Verfahren zur Herstellung von Acetylketendialkylacetalen der Formel $CH_3CO\text{-}CH=C(OR^2)_2$, wobei $R^2$ ein Alkylrest mit 1 bis 12 C-Atomen sein kann, dadurch gekennzeichnet, daß man einen Acetessigsäurealkylester der Formel $CH_3CO\text{-}CH_2COOR^1$ in Gegenwart eines sauren Katalysators mit einem gesättigten primären oder sekundären Alkohol der Formel $R^2OH$ umsetzt, wobei $R^2$ die angegebene Bedeutung hat und $R^2OH$ entweder mit $R^1OH$ identisch ist oder höher siedet als $R^1OH$.

2. Verfahren nach Anspruch 1, dadurch gekennzeichnet, daß man den Methyl- oder Ethylester der Acetessigsäure einsetzt.

3. Verfahren nach Anspruch 1 oder 2, dadurch gekennzeichnet, daß man, falls $R^2OH$ mit $R^1OH$ identisch ist, 2,5 bis 5 Mol $R^2OH$ pro Mol Acetessigsäurealkylester einsetzt.

4. Verfahren nach Anspruch 1 oder 2, dadurch gekennzeichnet, daß man, falls $R^2OH$ höher siedet als $R^1OH$, 1,5 bis 5 Mol $R^2OH$ pro Mol Acetessigsäurealkylester einsetzt.

5. Verfahren nach mindestens einem der Ansprüche 1 bis 4, dadurch gekennzeichnet, daß man einen primären Alkohol $R^2OH$ einsetzt, wobei $R^2$ ein Alkylrest mit 3 bis 6 C-Atomen ist.

6. Verfahren nach mindestens einem der Ansprüche 1 bis 5, dadurch gekennzeichnet, daß man bei einer Temperatur von 110 bis 170 °C arbeitet.

7. Verfahren nach mindestens einem der Ansprüche 1 bis 5, dadurch gekennzeichnet, daß man bei einer Temperatur von 30 bis 160 °C arbeitet.

8. Acetylketendialkylacetale der Formel $CH_3CO\text{-}CH=C(OR^2)_2$, wobei $R^2$ ein Alkylrest mit 3 bis 12 C-Atomen ist.

9. Acetylketendialkylacetale der Formel $CH_3CO\text{-}CH=C(OR^2)_2$, wobei $R^2$ ein Alkylrest mit 3 bis 8 C-Atomen ist.

## Claims

1. A process for the preparation of acetylketene dialkyl acetals of the formula $CH_3CO\text{-}CH=C(OR^2)_2$, in which $R^2$ can be an alkyl radical with 1 to 12 carbon atoms, which process comprises reacting an alkyl acetoacetate of the formula $CH_3CO\text{-}CH_2COOR^1$ in the presence of an acid catalyst with a saturated primary or secondary alcohol of the formula $R^2OH$, in which $R^2$ has the abovementioned meaning and $R^2OH$ is either identical with $R^1OH$ or is higher boiling than $R^1OH$.

2. The process as claimed in claim 1, wherein the methyl or ethyl ester of acetoacetic acid is employed.

3. The process as claimed in claim 1 or 2, wherein, in the case where $R^2OH$ is identical with $R^1OH$, 2.5 to 5 mol of $R^2OH$ per mol of alkyl acetoacetate are employed.

4. The process as claimed in claim 1 or 2, wherein, in the case where $R^2OH$ is higher boiling than $R^1OH$, 1.5 to 5 mol of $R^2OH$ per mol of alkyl acetoacetate are employed.

5. The process as claimed in at least one of claims 1 to 4, wherein a primary alcohol $R^2OH$ is employed, in which $R^2$ is an alkyl radical with 3 to 6 carbon atoms.

6. The process as claimed in at least one of claims 1 to 5, wherein the reaction is carried out at a temperature of 110 to 170°C.

7. The process as claimed in at least one of claims 1 to 5, wherein the reaction is carried out at a temperature of 130 to 160°C.

## Revendications

1. Procédé pour préparer des acétyl-cétène-dialkylacétals répondant à la formule: $CH_3COCH=C(OR^2)_2$, dans laquelle $R^2$ représente un radical alkyle contenant de 1 à 12 atomes de carbone, procédé caractérisé en ce qu'on fait réagir un acétyl-acétate d'alkyle de formule: $CH_3CO\text{-}CH_2COOR^1$ en présence d'un catalyseur acide, avec un alcool saturé primaire ou secondaire répondant à la formule: $R^2OH$ dans laquelle $R^2$ a la signification qui lui a été donnée ci-dessus, le composé $R^2OH$ étant identique à $R^1OH$ ou ayant un point d'ébullition supérieur à celui de $R^1OH$.

2. Procédé selon la revendication 1, caractérisé en ce qu'on utilise l'ester méthylique ou éthylique de l'acide acetyl-acétique.

3. Procédé selon l'une des revendications 1 et 2, caractérisé en ce que, dans le cas où $R^2OH$ est

identique à R$^1$OH, on utilise de 2,5 à 5 mol de R$^2$OH par mol de l'acétyl-acétate d'alkyle.

4. Procédé selon l'une des revendications 1 et 2, caractérisé en ce que, dans le cas où R$^2$OH a un point d'ébullition supérieur à celui de R$^1$OH, on utilise de 1,5 à 5 mol de R$^2$OH par mol de l'acétyl-acétate d'alkyle.

5. Procédé selon au moins l'une des revendications 1 à 4, caractérisé en ce qu'on utilise un alcool primaire R$^2$OH dans lequel R$^2$ représente un radical alkyle contenant de 3 à 6 atomes de carbone.

6. Procédé selon au moins l'une des revendications 1 à 5, caractérisé en ce qu'on opère à une température de 110 à 170°C.

7. Procédé selon au moins l'une des revendications 1 à 5, caractérisé en ce qu'on opère à une température de 130 à 160°C.